(19) Europäisches Patentamt
European Patent Office
Office européen des brevets

(11) Veröffentlichungsnummer : **0 110 281**
**B1**

(12) # EUROPÄISCHE PATENTSCHRIFT

(45) Veröffentlichungstag der Patentschrift : **10.09.86**

(51) Int. Cl.⁴ : **C 08 F 34/02**, C 12 N 11/08

(21) Anmeldenummer : **83111616.5**

(22) Anmeldetag : **21.11.83**

(54) **Vinylencarbonat-Polymerisate, Verfahren zu ihrer Herstellung und ihre Anwendung.**

(30) Priorität : **25.11.82 DE 3243591**

(43) Veröffentlichungstag der Anmeldung : **13.06.84 Patentblatt 84/24**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**DE-A- 2 014 242**
**DE-A- 2 407 340**
**FR-A- 2 335 532**
**GB-A- 1 009 004**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Mauz, Otto, Dr.**
**Heidestrasse 21**
**D-6237 Liederbach (DE)**
Erfinder : **Sauber, Klaus, Dr.**
**Falkenstrasse 35**
**D-6232 Bad Soden am Taunus (DE)**
Erfinder : **Noetzel, Siegfried, Dr.**
**An den Römergärten 3**
**D-6233 Kelkheim (Taunus) (DE)**

**EP 0 110 281 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, biologisch aktive Substanzen, wie Enzyme, Antikörper, Antigene, Hormone, unter Erhalt ihrer Aktivität an polymere Trägermaterialien über kovalente Bindungen zu fixieren, um auf diesem Weg beispielsweise Enzyme zu stabilisieren, zu reinigen oder wasserunlöslich zu machen. Solchermaßen immobilisierte biologisch aktive Substanzen bieten erhebliche Vorteile gegenüber der löslichen Form : Zum einen ist die Abtrennbarkeit durch Sedimentation nach Beendigung einer Reaktion vereinfacht, zum anderen ist die Stabilität und Wiederverwendbarkeit der Präparate um ein Vielfaches erhöht, da eventuell vorhandene Proteasen mit fixiert werden.

In der DE-AS 2 237 316 werden als Trägersubstanzen quellbare, vernetzte Perlpolymerisate beschrieben, die durch Copolymerisation von reaktiven Gruppen enthaltenden Monomeren, vernetzenden Monomeren und hydrophilen Monomeren erhalten werden. Als reaktive Gruppen werden dabei die Halogenalkyl-, die Epoxid-, die Carbonsäurechlorid-, Carbonsäureanhydrid-, Carbonsäureazid-, Carbonsäurephenylester- und Hydroxamsäure-Gruppe offenbart. Diese Trägermaterialien haben jedoch eine Reihe von Nachteilen ; so ist die Fixierung der biologisch aktiven Substanzen bei einigen von ihnen ziemlich langwierig und dauert häufig mehrere Tage ; bei Verwendung der Anhydridvarianten kommt es außerdem zur Einführung von Ladungen.

Gemäß der DE-OS 2 407 340 werden Polymere und Copolymere des Vinylencarbonats zur Bindung von biologisch aktiven Substanzen verwendet. Auch nach der DE-AS 2 552 510 können Copolymerisate des Vinylencarbonats für diesen Zweck eingesetzt werden. Die nach Fixierung der biologisch aktiven Substanz noch vorhandenen Cyclocarbonatgruppen werden nach einer Variante des darin beschriebenen Verfahrens in Hydroxygruppen übergeführt.

Die in diesen beiden Druckschriften offenbarten Polymerträger auf Basis von Vinylencarbonat als reaktive Monomereinheit haben zwar gegenüber anderen Trägermaterialien den Vorteil geringer unspezifischer Adsorption biologisch aktiver Substanzen, beispielsweise bei Einsatz als Affinitätsadsorbens ; außerdem sind sie leicht modifizierbar. Ihnen fehlt jedoch die perlförmige Morphologie und die erforderliche Porosität. Dadurch sind sie beispielsweise für Säulenverfahren nicht anwendbar.

Unregelmäßig geformte Copolymerisate des Vinylencarbonats fallen nach der in der DE-OS 2 556 759 beschriebenen Verfahrensweise an, wonach die Polymerisation in einem organischen unpolaren Dispergiermittel in Gegenwart von bestimmten nichtionogenen Dispersionsstabilisatoren erfolgt. Auch diese Polymerisate besitzen keine Perlform und auch nicht das notwendige Maß an Porosität.

Die Aufgabe vorliegender Erfindung bestand daher darin, ein polymeres Material, insbesondere Trägermaterial für biologisch aktive Substanzen auf Basis von Vinylencarbonat bereitzustellen, das die Nachteile des Standes der Technik nicht besitzt und insbesondere Perlform und eine ausreichende Porosität aufweist. Eine weitere Aufgabe bestand darin, ein hierfür geeignetes Verfahren zu entwickeln.

Zur Lösung dieser Aufgabe schlägt die Erfindung daher ein Polymerisat vor, bestehend aus Einheiten, die sich vom Vinylencarbonat oder dessen durch einen einwertigen Kohlenwasserstoffrest mit bis zu 8 Kohlenstoffatomen substituiertes Derivat und mindestens einem weiteren damit copolymerisierbaren Monomeren ableiten, wobei die mittlere Teilchengröße der Polymerteilchen im Bereich von 20 bis 800 µm liegt, dadurch gekennzeichnet, daß die Polymerteilchen im wesentlichen perlförmige Gestalt und einen mittleren Porendurchmesser von 5 bis 1 000 nm aufweisen.

Die Herstellung eines derartigen Perlpolymerisates erfolgt nach der Erfindung durch ein Verfahren, bei dem Vinylencarbonat oder ein durch einen einwertigen Kohlenwasserstoffrest mit bis zu 8 Kohlenstoffatomen substituiertes Derivat mit mindestens einem mit Vinylencarbonat copolymerisierbaren Monomeren in einem flüssigen Dispergiermedium polymerisiert wird, das unter den Polymerisationsbedingungen die Monomeren, das Polymerisat und vorzugsweise auch den Initiator nicht löst, in Gegenwart eines radikalisch wirksamen Initiators und eines Dispersionsstabilisators, dadurch gekennzeichnet, daß als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen $\alpha$-Olefin mit 8 bis 30 C-Atomen verwendet wird.

Die Erfindung hat weiterhin die Verwendung der so erhaltenen Polymeren als Trägermaterialien zur Herstellung von trägergebundener biologisch aktiver Substanzen zum Gegenstand.

Das erfindungsgemäße Perlpolymerisat enthält die sich vom Vinylencarbonat oder dessen Derivaten ableitenden Einheiten zweckmäßigerweise in Mengen von 5 bis 90 Mol.-%, vorzugsweise 10 bis 80 Mol.-%, bezogen auf das Gesamtpolymere. Größere und kleinere Mengen als die vorstehend angegebenen sind grundsätzlich möglich, jedoch ist dies im Regelfall mit Nachteilen verbunden. Die optimale Menge an sich vom Vinylencarbonat oder sich von dessen Derivaten ableitenden Einheiten innerhalb der vorstehend angegebenen Bereiche hängt u. a. ab von der angestrebten Besetzungsdichte, von dem Molekulargewicht der biologisch aktiven Substanz.

Vinylencarbonat ist erfindungsgemäß als Monomeres, das dem Perlpolymerisat reaktive Gruppen verleiht, bevorzugt. Bei dessen erfindungsgemäß ebenfalls einsetzbaren Derivaten handelt es sich um solche der Formel

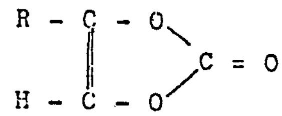

(I)

worin R für einen einwertigen Kohlenwasserstoffrest mit bis zu 8 Kohlenstoffatomen, vorzugsweise für einen Alkylrest mit 1 bis 6 Kohlenstoffatomen und insbesondere einen solchen mit 1 bis 4 Kohlenstoffatomen steht. Beispiele hierfür sind : der Methyl-, Äthyl-, Isopropyl-, 2-Äthylhexyl-, n-Heptyl-, Cyclopentyl-, Allyl-, Phenyl-, Tolyl-, Benzyl- oder Xylylrest.

Falls vor dem Einsatz als Trägermaterial eine Modifizierung mit sogenannten Spacern erfolgt (siehe hierzu weiter unten), so enthält das erfindungsgemäße Perlpolymerisat neben unveränderten Vinylencarbonat-Einheiten gemäß (I) bzw. Vinylendiol (1,2)-Einheiten (im Falle einer vorherigen Verseifung) auch entsprechende mit dem Spacer modifizierte Einheiten. Diese werden zumeist der nachstehenden Formel (II) entsprechen, d. h. es wird im Regelfall an benachbarten C-Atomen in der Polymerkette nur eine Spacereinheit vorhanden sein :

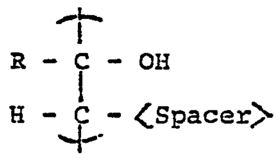

(II)

R hat hierzu die obige Bedeutung und « Spacer » steht insbesondere für

A—B—C

worin A gleich O oder OCONH ist, B für einen organischen Rest (den Spacer im engeren Sinne), insbesondere für einen Kohlenwasserstoffrest mit 1 bis 12 C-Atomen steht, der gegebenenfalls durch Heteroatome wie O, NH, S unterbrochen sein kann, und C eine funktionelle Gruppe darstellt, die mit der biologisch aktiven Verbindung eine kovalente Bindung eingehen kann. Hierzu gehören beispielsweise die Gruppen COOH, $NH_2$

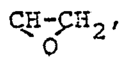

COX (X = H, Halogen, $N_3$, OR ; R = Alkylrest mit 1 bis 6 C-Atomen), $CH(OR)_2$ (R wie vorstehend),

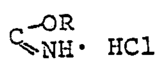

(R wie vorstehend), $N_2$ oder NCO.

Vorzugsweise bedeutet A gleich O, B einen aliphatischen, insbesondere unverzweigten Kohlenwasserstoffrest mit 1 bis 6 C-Atomen, einen Arylrest oder einen Alkylarylrest und C gleich $NH_2$,

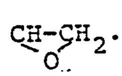

Beispiele für die Gruppierung A—B—C sind :

$-O-(CH_2)_n-NH_2$ ; n = 2-12

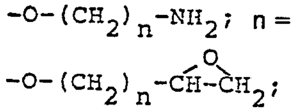 n = 1-8

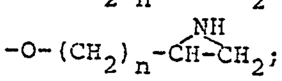 n = 1-8

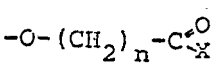 n = 1-8
X = H, OH, Halogen, $N_3$, OR

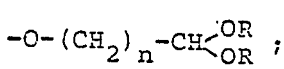 n = 1-6
R = Alkylrest mit 1-6 C-Atomen

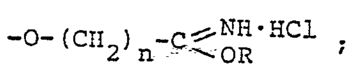 n = 2-8
R = Alkylrest mit 1-6 C-Atomen

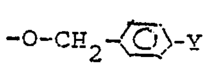 Y = $NH_2$, $N_2$, NCO

Die Menge angegeben in Mol-% an Monomereinheiten gemäß (II) bzw. an Spacern, bezogen auf alle sich vom Vinylencarbonat herleitenden Einheiten, beträgt bis zu $Y_1$ Mol-%, insbesondere $Y_2$ Mol-% wobei

$$Y_1 = 91 — 0{,}007\ 53\ X^2 \text{ und}$$

$$Y_2 = 95 — 0{,}94\ X$$

betragen und X die Anzahl Mol-% aller sich vom Vinylencarbonat ableitenden Einheiten in Bezug auf die Gesamtmonomeren ist. Die Mindestmenge, angegeben in Mol-%, an Monomereinheiten gemäß (II) bzw. an Spacern beträgt 0,01 Mol-%, bevorzugt 0,5 Mol-%, bezogen auf die Gesamtmonomeren. Im allgemeinen wird die Höchstmenge an diesen Monomereinheiten etwa 35 Mol-%, bezogen auf die Gesamtmonomeren, nicht übersteigen.

Das Perlpolymerisat enthält erfindungsgemäß noch mindestens eine weitere Monomereinheit, die sich von einem mit Vinylencarbonat copolymerisierbaren Monomeren ableitet. Vorzugsweise handelt es sich hierbei um zwei weitere voneinander verschiedene Monomereinheiten.

Eine davon ist vorzugsweise ein Monomeres mit hydrophilen Gruppen, welche dem als Träger eingesetzten Polymeren ausreichende Hydrophilie und damit Quellbarkeit verleiht. Dies ist insofern von Bedeutung, als die Verankerungsreaktion mit der biologisch aktiven Substanz zumeist im wässrigen System durchgeführt wird und die hydrophile biologisch aktive Substanz an das Trägermaterial herandiffundieren können muß. Zu diesen Monomeren gehören beispielsweise die in der DE-OS 2 237 316 als Komponente c) aufgeführten Monomeren. Im Rahmen der vorliegenden Erfindung werden dabei bevorzugt : N-Vinylpyrrolidon, (Meth)-Acrylsäure, (Meth)-Acrylamid, (Meth)-Acrylsäurealkylester mit jeweils 2 bis 4 C-Atomen in der Alkylgruppe, Hydroxyalkylester der (Meth)acrylsäure mit 2 bis 6 C-Atomen in der Alkylgruppe, N-Vinyl-N-alkylacetamid ($C_1$-$C_4$-Alkyl) und Vinylacetat. Gegebenenfalls können auch mehrere dieser hydrophilen Monomer-Einheiten vorhanden sein.

Das Verhältnis von Vinylencarbonat zu hydrophiler Komponente ist dabei auch abhängig von der Art des zu bindenden Enzyms. Bei sehr hohen Molekulargewichten des Enzyms bzw. des Substrates, das mit dem Enzym reagieren soll, ist es zweckmäßig, das Molverhältnis zu Gunsten der hydrophilen Komponente zu erhöhen, da aus sterischen Gründen benachbarte bindungsfähige Carbonatgruppen nicht zur Reaktion gelangen, evtl. sogar störend wirken können. Weiterhin richtet sich die Menge an hydrophiler Komponente auch nach der Menge der erfindungsgemäß bevorzugt vorhandenen vernetzenden Komponente. Je größer deren Menge ist, desto größer wird in der Regel auch die erforderliche Menge an hydrophiler Komponente sein, um dem Trägerpolymeren ausreichende Hydrophilie und Quellbarkeit zu vermitteln. Im allgemeinen beträgt erfindungsgemäß die Menge an hydrophiler(n) Monomer-Einheit(en) im Polymeren 5 bis 70 Mol.-%, vorzugsweise 20 bis 50 Mol.-%, bezogen auf das Polymere.

Als weitere mit den Vinylencarbonat copolymerisierbare Verbindung enthält das erfindungsgemäße Polymere bevorzugt vernetzende Monomer-Einheiten, wie sie aus dem Stand der Technik bekannt sind und beispielsweise in der DE-OS 2 237 316 als Komponente b) beschrieben sind. Als typische Vertreter seien hier genannt : Divinyläther von Glykolen wie Äthylenglykoldivinyläther, Butandioldivinyläther, Hexandiol-divinyläther, N,N'-Methylenbisacrylamid, N,N'-Methylenbis-methacrylamid, Äthylenglykol-bismethacrylsäureester, Butandiolbismethacrylsäureester, Triallylcyanurat, Trisacryloylperhydrotriazin, Divinylbenzol, Adipinsäuredivinylester, N,N'-Divinyläthylenharnstoff, Vinylacrylat, Allylmethacrylat u. a. Es können auch mehrere verschiedene vernetzende Monomereinheiten vorhanden sein. Einzelne dieser Vernetzer, z. B. der N,N'-Divinylethylenharnstoff oder das N,N'-Methylenbisacrylamid können auch zur Hydrophilie des Polymeren beitragen.

Die Menge an vernetzender Monomer-Einheit und damit die Vernetzungsdichte hängt dabei von der Anwendung ab. Bei Enzymreaktionen im Rührkessel oder für Diagnostika kann eine geringe Vernetzungsdichte vorteilhaft sein ; wird dagegen eine Säulenfüllung vorgenommen, ist eine hohe Formstabilität der Perlpolymerisate und damit eine hohe Vernetzungsdichte Voraussetzung. Je nach Anwendungsart kann daher die Menge an vernetzender Monomer-Einheit bis zu 60 Mol.-%, bezogen auf das Polymere, betragen. Vorzugsweise liegt sie zwischen 5 und 40 Mol.-%. Wie hierzu bereits weiter oben ausgeführt, steht die Menge an vernetzender Komponente mit der der hydrophilen Komponente in gewisser Relation. Zumeist wird man die Menge an vernetzendem Monomeren so wählen, daß das Perlpolymerisat in Tetrahydrofuran bis auf das 14-fache, vorzugsweise das 0- bis 8-fache seines ursprünglichen Schüttvolumens aufquillt.

Unvernetzte Träger sind dann von Interesse, wenn die Umsetzung des Trägers mit der biologisch aktiven Substanz in einer Lösung des Trägers vorgenommen werden soll.

Gegebenenfalls kann ein unvernetztes Trägerpolymeres in bekannter Weise auch durch nachträgliche chemische Umsetzung vernetzt werden, z. B. durch Diamine. Das Polymere sollte in diesem Fall einen etwas höheren Anteil an Vinylencarbonatgruppen enthalten.

Als gegebenenfalls weitere nicht hydrophile und nichtvernetzende Monomer-Einheiten können beispielsweise solche vorhanden sein, die sich ableiten von : Acryl- und Methacrylsäureester mit 5-12 C-Atomen im Alkylrest, (Meth)-Acrylnitril, Vinylester mit 4-18 C-Atomen in der Carbonsäure, wie Vinylbutyrat, Vinylstearat und Vinylester verzweigter Carbonsäuren mit 10-12 C-Atomen ; weiterhin Vinylaromaten, wie Styrol oder α-Methylstyrol. Diese Monomer-Einheiten können im Polymeren in Mengen von 4 bis 40 Mol.-%, vorzugsweise 8 bis 20 Mol.-%, bezogen auf das Polymere, vorhanden sein.

Das erfindungsgemäße Perlpolymere besteht aus überwiegend kugelförmigen Teilchen, deren mittlere Teilchengröße im trockenen, ungequollenen Zustand 20 bis 800 µm, vorzugsweise 50 bis 300 µm beträgt und die vorzugsweise eine enge Teilchengrößenverteilung aufweisen. Das jeweilige Optimum der Teilchengröße hängt dabei vor allem von dem speziellen Einsatzgebiet ab. Bei einem ohne Druck durchgeführten Säulenverfahren wird man beispielsweise die Teilchengröße innerhalb der vorstehend genannten Grenzen entsprechend größer wählen können als bei einem Druck-Verfahren. Die Perlen des erfindungsgemäßen Perlpolymerisates sind überwiegend als Hohlperlen ausgebildet, was eine hohe Porosität bedingt. Dies drückt sich in dem sich daraus ergebenden erfindungsgemäßen mittleren Porendurchmesser im Bereich von 5 bis 1 000 nm, vorzugsweise 10 bis 500 nm und insbesondere 20 bis 200 nm aus.

Die Bestimmung des Porendurchmessers (Porenvolumens) erfolgt in der Weise, daß zunächst das Porenvolumen gemäß der Kapillardruckmethode (Quecksilberporosimetrie) bestimmt wird (vgl. hierzu « Ullmanns Encyklopädie der technischen Chemie », Bd. 5 (1980), S. 751-752). Daraus ergibt sich dann der mittlere Porendurchmesser durch Berechnung nach der auf Seite 752, linke Spalte oben dieser Literaturstelle angegebenen Gleichung.

Die erfindungsgemäßen Perlpolymerisate eignen sich insbesondere als Träger für biologisch aktive Substanzen. Sie können aber auch für andere Zwecke eingesetzt werden, beispielsweise als Ionenaustauscher, Adsorptionsmittel für chromatographische Verfahren.

Das erfindungsgemäße Verfahren zur Herstellung dieser Perlpolymeren wird unter den üblichen bekannten Bedingungen der Perlpolymerisation durchgeführt, wie sie beispielsweise in der DE-OS 2 237 316 oder der DE-OS 2 556 759 beschrieben sind, jedoch mit der Neuerung, daß spezielle Dispersionsstabilisatoren zum Einsatz kommen.

Bei diesen handelt es sich um vorzugsweise alternierende Copolymere aus Maleinsäureanhydrid und einem Vinylalkyläther, vorzugsweise einem Vinyl-n-alkyläther mit 6 bis 30 C-Atomen, vorzugsweise 10 bis 20 C-Atomen in der Alkylgruppe, oder einem Vinylester mit 6 bis 30 C-Atomen, vorzugsweise 10 bis 20 C-Atomen in der Carbonsäuregruppe oder einem längerkettigen α-Olefin mit 8 bis 30 C-Atomen, vorzugsweise 10 bis 20 C-Atomen. Als Beispiele für derartige Vinylalkyläther, Vinylester bzw. längerkettigen α-Olefinen seien hier genannt: Vinyloctyläther, Vinyldecyläther, Vinyldodecyläther, Vinylstearyläther, Vinylmyricyläther, Ethylhexansäure-Vinylester, Isononansäure-Vinylester, Versatiesäure-Vinylester, Laurinsäure-Vinylester, Stearinsäure-Vinylester und Vinylester verzweigter Carbonsäuren mit 10 bis 12 C-Atomen ; Octen-(1), Decen-(1), Dodecen-(1), Tetradecen-(1), Octadecen-(1), Tricosen-(1).

Diese Dispersionsstabilisatoren sind bereits in Mengen von 0,001 Gew.-%, bezogen auf die Gesamtmenge an Monomeren, wirksam. Zumeist werden Mengen von 0,005 bis 10 Gew.-%, vorzugsweise 0,01-5 Gew.-% (bezogen auf Gesamtmenge an Monomeren) verwendet.

Die reduzierte spezifische Viskosität (RSV) dieser als Dispersionsstabilisatoren eingesetzten Copolymeren liegt in der Regel zwischen 0,01 und 1,0 [dl/g], (bestimmt in 0,6 %iger Lösung in Toluol bei 25 °C). Der entsprechende Vorzugsbereich beträgt bei den Copolymeren aus Maleinsäureanhydrid und Vinylalkyläther bzw. Vinylester 0,05 bis 1,0 [dl/g] und bei den Copolymeren aus Maleinsäureanhydrid und längerkettigem α-Olefin 0,01 bis 0,1 [dl/g]. Das Molverhältnis zwischen Maleinsäureanhydrid und dem Vinylalkyläther bzw. Vinylester oder dem längerkettigen α-Olefin liegt vorzugsweise bei 1 : 1.

Als radikalisch wirksame Initiatoren kommen erfindungsgemäß solche in Betracht, die in der Monomerphase gut und in dem flüssigen Dispergiermedium möglichst wenig löslich sind. Beispiele hierfür sind organische Peroxide, wie Di-tert.-butylperoxid, Dibenzoylperoxid, Cumolhydroperoxid, Cyclohexanonperoxid oder aliphatische Azoverbindungen, wie α,α'-Azodiisobuttersäurenitril, Azo-biscyanvalerinsäure, 1,1'-Azo-cyclo-hexan-1,1'-dicarbonsäuredinitril und Azodicarbonamid. Gegebenenfalls können auch entsprechende Redoxsysteme Verwendung finden. Die Menge an Initiator beträgt zumeist 0,01-5, vorzugsweise 0,1-2 Gew.-% (bezogen auf die Gesamtmenge der Monomeren).

Als flüssige Dispergiermedien zur Durchführung der erfindungsgemäßen Perlpolymerisation dienen vor allem solche organischen Verbindungen, die unter Normalbedingungen flüssig sind, einen Siedepunkt von oberhalb 60 °C, vorzugsweise im Bereich von 85-300 °C, aufweisen und welche die Monomeren, das Polymere und vorzugsweise auch den Initiator unter den Polymerisationsbedingungen nicht oder jedenfalls nur spurenweise lösen, um die unerwünschte Fällungspolymerisation zu unterbinden. Gut geeignet sind beispielsweise Kohlenwasserstoffe mit 6-20, vorzugsweise 12-16 Kohlenstoffatomen, insbesondere Paraffine. Auch ein Gemisch von verschiedenen Verbindungen kann als Dispersionsmittel eingesetzt werden. Geeignete Kohlenwasserstoffe oder Kohlenwasserstoffgemische sind z. B. n-Hexan, n-Heptan, n-Oktan, Cyclohexan, iso-Oktan, Benzinfraktionen mit Siedebereichen zwischen 90 und 170 °C und Paraffinöl dünnflüssig (Deutsches Arzneibuch, 7. Ausgabe, DAB 7). Das Verhältnis der Monomerphase zur Dispergiermittelphase kann in weiten Grenzen variieren, beispielsweise zwischen 0,5 : 1 bis 1 : 50, vorzugsweise 1 : 1 bis 1 : 15 (Gewichtsverhältnis).

Um eine möglichst hohe Porosität der Perlpolymerisate zu erreichen, werden dem Polymerisationssystem oder vorzugsweise den Monomeren vorzugsweise bestimmte inerte, flüssige Komponenten zugesetzt. Hierunter sollen solche Stoffe verstanden sein, in denen sich die Monomeren gut lösen oder die sich in den Monomeren gut auflösen oder mit ihnen mischbar, andererseits aber im Dispergiermedium praktisch unlöslich und damit mit diesem nicht mischbar sind. Nach ihrem Verhalten zu den entsprechenden Copolymeren kann man die inerten Komponenten in Quellungs- und/oder Fällungsmittel

einteilen. Bei einer hydrophilen Matrix werden polare Inertmittel in der Regel eine Quellung begünstigen, wie z. B. Dimethylformamid, Dimethylsulfoxid, Dioxan, Wasser in gewissem Umfang, während sich unpolare Substanzen, wie Glycerintriacetat etc. als Fällungsmittel für das Copolymerisat erweisen. Dasselbe gilt auch für den umgekehrten Fall, d. h. bei hydrophoben Polymeren werden unpolare Lösungsmittel als Quellmittel dienen. Das optimale Inertmittel bzw. Inertmittelgemisch läßt sich durch einige einfache Routineversuche leicht ermitteln. Insbesondere, wenn Perlpolymere mit relativ geringem Vernetzungsgrad angestrebt werden, dürfte es sich empfehlen, eine Mischung aus polarem und unpolarem Inertmittel einzusetzen. Die inerten Komponenten nehmen an der Polymerisation nicht teil, werden jedoch vom Polymerisat umhüllt und bei der Aufarbeitung wieder herausgelöst. Dadurch entstehen permanente Poren. Die Porengröße ist durch Art und Menge der Inertkomponente beeinflußbar, hängt aber auch von der Menge an vernetzender Komponente ab.

Die Inertkomponenten können allein oder in Mischung eingesetzt werden. Als Beispiele seien genannt: Methanol und seine höheren Homologen, Äthylenglykol, Methylglykol, Propylglykol, Diäthylenglykol, Butandiol-(1,4), Glycerin, Polyäthylenglykole, Diäthylenglykol-dimethylether, Glycerintriacetat, Ethylencarbonat, Formamid, Dimethylformamid, Dimethylsulfoxid, Dioxan, Wasser in gewissem Umfang.

Die Menge an zugesetzter Inertkomponente ist weitgehend variabel. Sie hängt u. a. von der Monomerzusammensetzung des Trägers, insbesondere seines Gehaltes an Vernetzer, der erwünschten Porosität (Porengröße) sowie vom genauer Verwendungszweck des Trägerpolymeren ab. So wird sich bei einem hohen Vernetzungsgrad eine entsprechend große Menge an Inertkomponente empfehlen, um eine bestimmte Porosität (Porengröße) zu erreichen. Bei ein und demselben Vernetzungsgrad wird die Porosität (Porengröße) gleichfalls umso größer sein, je mehr an Inertkomponente eingesetzt wird. Naturgemäß läßt sich dies nur innerhalb bestimmter Grenzen steigern, da ansonsten die Wandstärke der Hohlperlen und damit deren mechanische Festigkeit zu gering wird. In den meisten Fällen wird eine Menge an Inertmittel, die dem 0,02- bis 5-fachen, vorzugsweise dem 0,04- bis 3-fachen der Menge an eingesetzten Monomeren entspricht, zufriedenstellende Ergebnisse liefern.

Das Vinylencarbonat sowie das weitere (bzw. die weiteren) Comonomere(n) werden in solchen Mengen eingesetzt, daß ein Polymeres mit den weiter oben angegebenen Mengen an Monomer-Einheiten resultiert. Hierzu wird das Vinylencarbonat in der Regel in Mengen von 5 bis 95 Mol.-%, vorzugsweise 10 bis 85 Mol.-%, bezogen auf das Gesamtmonomerengemisch verwendet. Die Menge an hydrophilen Monomeren beträgt demgegenüber zumeist 3 bis 60 Mol.-%, vorzugsweise 15 bis 35 Mol.-%, bezogen auf das Gesamtmonomerengemisch, und die Menge an vernetzendem Monomeren — falls eingesetzt — bis zu 60 Mol.-%, vorzugsweise 2 bis 35 Mol.-%, bezogen auf die Gesamtmonomerenmenge.

Das erfindungsgemäße Verfahren wird zweckmäßigerweise in einem mit einer Rührvorrichtung versehenen Reaktionsgefäß bei Temperaturen von zumeist 20-150 °C, vorzugsweise 65-125 °C durchgeführt. Die Teilchengröße des Perlpolymerisates wird in bekannter Weise durch die Rührgeschwindigkeit und das Phasenverhältnis eingestellt. Besonders vorteilhaft ist die Verwendung einer senkrecht stehenden, zylindrischen Gefäßes mit flachem Boden, das mit einem koaxial angebrachten Rührer versehen ist, dessen Welle bis fast auf den Gefäßboden reicht. Das Reaktionsgefäß ist vorzugsweise vakuumfest und kann mit Rückflußkühler, Zulauftrichter, Gaseinleitungsrohr und Temperaturmeßgerät versehen werden. Die Beheizung und Kühlung des Gefäßes erfolgt im allgemeinen durch ein Flüssigkeitsbad, z. B. ein Ölbad oder Wasserbad.

Es ist vorteilhaft, das erfindungsgemäße Verfahren unter Ausschluß von Luftsauerstoff durchzuführen. Das Reaktionsgefäß wird daher vor Beginn mit einem inerten Gas, vorzugsweise Stickstoff, gespült.

Nach Beendigung der Polymerisationsreaktion werden die nicht umgesetzten Monomeren aus dem Reaktionsgefäß entfernt, z. B. durch Verdampfen bei vermindertem Druck, vorzugsweise bei einem Druck von 0,13-20 mbar (0,1-15 Torr). Nach der Entfernung der Restmonomeren wird das Dispersionsmittel vom festen Polymeren abgetrennt, z. B. durch Dekantieren, Filtrieren oder Absaugen des Überstandes. Anschließend wird das Polymerisat, falls erforderlich, mit leichtsiedenden organischen Lösungsmitteln, z. B. einem Kohlenwasserstoff, einem niederen Alkohol oder Aceton gewaschen und schließlich getrocknet. Die Trocknung des Polymerisates erfolgt bei einer Temperatur von zumeist 20-100 °C, vorzugsweise von 40-80 °C ; eine Trocknung unter vermindertem Druck ist dabei empfehlenswert.

Bei der mit den erfindungsgemäßen Perlpolymeren zu verankernden biologisch aktiven Substanz handelt es sich um die bekannten *in vivo* oder *in vitro* wirksamen natürlichen oder künstlich hergestellten Stoffe, wie Enzyme, Aktivatoren, Inhibitoren, Antigene, Antikörper, Vitamine, Hormone, Effektoren, Antibiotika, Proteine. Der letzere Begriff umfaßt dabei auch Proteine mit bestimmten Nicht-Proteinsubstituenten wie Metallionen, Polysacchariden, Porphyringruppen, Adenindinucleotid, Ribonucleinsäure, Phospholipide etc. Auch Polypeptidfragmente, z. B. die aktiven Teile von Enzymmolekülen, fallen unter den Begriff biologisch aktive Substanzen.

Von den vorstehend genannten biologisch aktiven Substanzen sind erfindungsgemaß die Enzyme bevorzugt.

Beispiele für Enzyme sind Adenyldesaminase, Alkohol-Dehydrogenase, Asparaginase, Carboxypeptidase, Chymotrypsin, Diphosphoesterase, -Glucosidase, Glucose-Isomerase, Glucose-Oxidase, Glucose-6-phosphat-Dehydrogenase, Hexokinase, Invertase, β-Lactamase, Lactase, Lactat-Dehydrogenase, versch. Lectine, NAD-Kinase, Neuraminidase, Papain, Peroxidase, Phosphatasen (alkalisch und sauer), 5'-Phosphodiesterase, Pyruvat Kinase, Ribonuclease, Trypsin.

Beispiele für andere biologisch aktive Substanzen sind Hormone, wie Insulin und die verschiedensten Hypophysen-Hormone, Proteine der gamma-Globulinfraktion, z. B. Antikörper der Klasse G, M, A, D und E, andere Blutfaktoren, z. B. der Antihämophiliefaktor, die Blutgerinnungsfaktoren, spezielle Antikörper, z. B. Hepatitis-, Poliomyelitis-, Finnen-, Mumps-, Influenza- oder Kaninchenantikörper, Antigene, wie Hepatitis-, Polyomyelitis-, Finnen-, Mumps-, Influenza- oder Kaninchenantigen zur Reinigung oder Stimulierung geeigneter Antikörperreaktionen, wobei das Antigen (nach dem Unlöslichmachen) in der unlöslichen Form verbleibt und folglich nicht in den Körper eindringen und diesen schädigen kann, sowie allgemeine Körperproteine, wie Hämoglobin oder Albumin.

Die Verankerungsreaktion zwischen der biologisch aktiven Substanz wird in bekannter Weise durchgeführt, wie etwa in der DE-OS 2 407 340 oder in den DE-PSen 2 215 687, 2 421 789 und 2 552 510 beschrieben. Zumeist wird die Umsetzung bei Raumtemperatur oder darunter liegenden Temperaturen durchgeführt. Letzteres insbesondere dann, wenn die zu verankernde biologisch aktive Substanz von Hause aus instabil ist ; in diesem Fall liegen dann die Temperaturen unterhalb von + 10 °C, vorzugsweise bei 0 bis + 5 °C.

Die Verankerungsreaktion erfolgt vorzugsweise in der Umgebung eines neutralen pH-Wertes, beispielsweise bei pH-Werten von 5-9, da hier die meisten biologisch aktiven Substanzen am stabilsten sind. In der Regel ist es auch nicht erforderlich, stärker saure oder alkalische Bedingungen einzuhalten, da die erfindungsgemäßen makroporösen Perlpolymerisate auch bereits im Neutralbereich mit den meisten der in Frage kommenden Substanzen schnell reagieren. Die dabei entstehende Bindung bietet genügend Stabilität für lange Lagerungen und hohe Operationsstabilität.

Als Träger kann dabei unmittelbar das Vinylencarbonatgruppen enthaltende Polymere oder das Verseifungsprodukt verwendet werden. In vielen Fällen wird es aber zweckmäßig sein, das mit sog. « Spacern » modifizierte Polymer einzusetzen. Unter « Spacer » versteht man dabei Verbindungen, die sowohl mit dem Trägerpolymeren als auch mit der biologisch aktiven Substanz reagieren und zwischen beiden gewissermaßen eine Brücke bilden. Die Umsetzung des erfindungsgemäßen Perlpolymerisates mit dem Spacer kann entweder direkt oder vorzugsweise nach vorheriger Verseifung der Carbonatgruppen erfolgen. Der Umsatzgrad hängt dabei u. a. von der Sperrigkeit des Spacers und der Zugänglichkeit der Carbonatgruppe bzw. der daraus entstandenen sekundären Hydroxylgruppen ab. Als Spacer kommen erfindungsgemäß die hierfür bekannten homo- und hetero-bifunktionellen Verbindungen in Frage, deren zweite funktionelle Gruppe die Kopplung mit der zu fixierenden biologisch aktiven Substanz übernimmt (vgl. die DE- Patentschrift 2 421 789 und 2 552 510, sowie Ullmanns Encyclopädie der technischen Chemie, 4. Auflage, Bd. 10, S. 540 und « Characterization of Immobilized Biocatalysts », Verlag Chemie, Weinheim, 1979, S. 53).

Bei der erfindungsgemäß bevorzugten Spacern handelt es sich um solche, welche die nachstehenden Gruppen einführen :

$-O-(CH_2)_n-NH_2$ ; n = 2-12

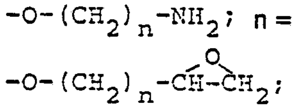 n = 1-8

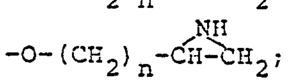 n = 1-8

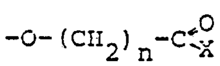 n = 1-8
X = H, OH, Halogen, $N_3$, OR

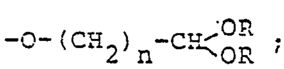 n = 1-6
R = Alkylrest mit 1-6 C-Atomen

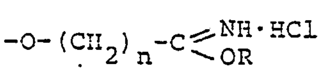 n = 2-8
R = Alkylrest mit 1-6 C-Atomen

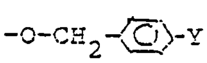 Y = $NH_2$, $N_2$, NCO.

Gemäß einer so erfindungsgemäß bevorzugten Ausführungsform wird das Perlpolymere nach Verseifung der Carbonatgruppen beispielsweise mit Epichlorhydrin umgesetzt.

Pro 1 Gewichtsteil der zu verankernden biologisch aktiven Substanz werden vorzugsweise etwa 10 bis 80 Gewichtsteile des Trägerpolymeren, insbesondere eines solchen, in welchem mindestens 50 Mol.-% Vinylencarbonateinheiten enthalten sind, verwendet. Größere Mengen des Trägerpolymeren können zweckmäßigerweise in den Fällen eingesetzt werden, n denen die Vinylencarbonateinheiten weniger als 50 Mol.-% des Polymeren ausmachen.

Die Erfindung wird nachfolgend anhand von Beispielen näher erläutert.

Herstellung eines Dispersionsstabilisators (Copolym. aus Maleinsäureanhydrid und Vinylstearyläther)

In einem Rührkolben wurden 98 g Maleinsäureanhydrid (1 Mol) und 296 g Vinylstearyläther (1 Mol) in 250 ml Aceton vorgelegt, 5 ml Diisopropylperkarbonat (40 %-ige Lösung in Phthalat) zugesetzt und das Gemisch unter Rühren und Stickstoff 5 Stunden bei 60° polymerisiert. Nach Abkühlen wurde das ausgefallene Produkt abgesaugt und mehrmals mit Aceton gewaschen.

Das Molverhältnis der beiden Monomeren im Copolymeren betrug 1 : 1 ; der RSV-Wert lag bei 0,224 [dl/g] (gemessen in 0,6 %iger Lösung in Toluol bei 25 °C).

I. Herstellung von perlförmigen Vinylencarbonat-Polymerisaten.

Beispiel 1

(Copolymerisat aus Vinylencarbonat, N-Vinylpyrrolidon und Butandiol-1,4-divinyläther)

In einem Rundkolben mit Rührer, Thermometer, Stickstoffeinleitrohr und Rückflußkühler wurden 900 ml dünnflüssiges Paraffinöl DAB 7 (Dispergiermedium), 0,8 g eines Copolymerisates aus Maleinsäureanhydrid und Octadecen-(1), Molverhältnis 1 : 1 ; RSV-Wert 0,064 [dl/g] (gemessen in 0,6 %iger Lösung in Toluol bei 25 °C), 4 ml Polyethylenglykol (Molekulargewicht etwa 400), 43 g Vinylencarbonat, 55,5 g N-Vinylpyrrolidon, 5 g Butandiol-1,4-divinylether und 2 g Azo-diisobuttersäurenitril vorgelegt. Das hier und für die nachfolgenden Beispiele benutzte Vinylencarbonat wurde vor dem Einsatz über eine 1 m lange, mit Raschig-Glasringen gefüllte Silbermantel-Kolonne bei 75°/44 mbar (33 Torr) überdestilliert. Das destillierte Vinylencarbonat wurde in dunklen Glasflaschen über Stickstoff im Kühlraum bei ca. 10° gelagert.

Das vorliegende Gemisch wurde dann unter Rühren langsam hochgeheizt. Bei ca. 65° begann die exotherme Polymerisationsreaktion, wobei die Temperatur auf ca. 80° anstieg. Diese Temperatur wurde mittels eines thermostatisierten Ölbades 2 Stunden gehalten, dann wurde zur Auspolymerisation die Badtemperatur eine Stunde auf 90° erhöht. Anschließend wurde das Heizbad entfernt und der Ansatz unter Rühren auf 40° abkühlen gelassen. Danach wurde die Rührung abgestellt, worauf sich nach einiger Zeit das perlförmige Polymerisat absetzte. Die Hauptmenge des Paraffinöls wurde dann abgehebbert und anschließend der Rest über eine Nutsche abgesaugt. Das erhaltene Polymerisat wurde anschließend unter Rühren mit Petrolether behandelt, um das anhaftende Paraffinöl zu entfernen. Anschließend wurde mit Methanol, dann mit Aceton ausgerührt, um die nicht umgesetzten Monomeren sowie den Dispergator herauszulösen. Schließlich wurde das Polymerisat im Vakuumtrockenschrank über Nacht bei 50-60° getrocknet.

Die Ausbeute betrug 82,8 g vernetztes Copolymerisat (= 80 % der Theorie).

Im wesentlichen das gleiche Produkt wird erhalten, wenn ein entsprechendes Dispergiermittel aus Maleinsäureanhydrid und Dodecen eingesetzt wird.

Beispiel 2

Copolymerisat aus Vinylencarbonat, N-Vinylpyrrolidon und Butandiol-1,4-divinylether.

In einem zylindrischen Gefäß, mit Kreuzbalkenrührer, Rückflußkühler, Thermometer und Stickstoffeinleitrohr wurden 900 g Paraffinöl (DAB 7), 0,2 g des unter a) beschriebenen Copolymerisates aus Maleinsäureanhydrid und Vinylstearylether 4 ml Polyethylenglykol 400, 43 g Vinylencarbonat, 55,5 g N-Vinylpyrrolidon, 20 g Butandiol-1,4-divinylether und 2 g Azo-diisobuttersäurenitril vorgelegt. Unter Einleiten von Stickstoff wurde die Badtemperatur langsam auf 65° hochgeheizt, dann wie im Beispiel 1 beschrieben polymerisiert und aufgearbeitet.

Die Ausbeute betrug 92,4 g vernetztes Produkt (= 78 % der Theorie).

Ein im wesentlichen gleiches Produkt wird erhalten, wenn als Dispergiermittel ein entsprechendes Copolymerisat aus Maleinsäureanhydrid und Vinyldodecylether eingesetzt wird.

Beispiel 3

Copolymerisat aus Vinylencarbonat, Acrylamid und Butandiol-1,4-divinylether.

In der in Beispiel 1 beschriebenen Apparatur wurde unter den in diesem Beispiel 1 beschriebenen Bedingungen folgendes System der Polymerisation unterworfen :

700 ml Paraffinöl, 1,8 g des in Beispiel 2 verwendeten Copolymerisats aus Maleinsäureanhydrid und Vinylstearylether, 114,6 g Vinylencarbonat, 47,3 g Acrylamid, 16,2 g Butandiol-1,4-divinylether, 1,8 g Azo-diisobuttersäurenitril und 100 ml Ethylenglykol.

Die Aufarbeitung erfolgte gleichfalls wie in Beispiel 1 beschrieben.

Die Ausbeute betrug 110 g vernetztes Copolymerisat (= 62 % der Theorie).

## Beispiel 4

Copolymerisat aus Vinylencarbonat, N-Vinylpyrrolidon und Ethylenglykoldimethylacrylat

In der Apparatur gemäß Beispiel 2 wurde unter den Bedingungen von Beispiel 1 folgendes System der Polymerisation unterworfen :

800 ml Paraffinöl, 0,18 g Copolymerisat aus Maleinsäureanhydrid und Octadecen-(1) gemäß Beispiel 1, 103,2 g Vinylencarbonat, 44,4 g N-Vinylpyrrolidon, 29,5 g Ethylenglykoldimethacrylat, 1,8 g Azo-diisobuttersäurenitril, 10 ml Polyethylenglykol (Molgewicht 400).

Die Aufarbeitung erfolgte wie in Beispiel 1 beschrieben.

Ausbeute : 120 g (67 % der Theorie).

## Beispiel 5

Copolymerisat aus Vinylencarbonat, Vinylacetat und Butandiol-1,4-divinylether

In der Apparatur gemäß Beispiel 2 wurde unter den Bedingungen von Beispiel 1 folgendes System der Polymerisation unterworfen :

900 ml Isooctan, 1,25 g Copolymerisat aus Maleinsäureanhydrid und Vinylstearylether (wie in Beispiel 2), 43 g Vinylencarbonat, 43 g Vinylacetat, 12 g Butandiol-1,4-divinylether, 1,3 g Azo-diisobuttersäurenitril, 20 ml Polyethylenglykol (Molgewicht 400).

Die Aufarbeitung erfolgte wie in Beispiel 1 mit Ausnahme der Hexanwäsche, die hier wegfiel.

Ausbeute : 52 g = 53 % der Theorie.

## Beispiel 6

Copolymerisat aus Vinylencarbonat, Vinylacetat und Butandiol-1,4-divinylether.

In der Apparatur gemäß Beispiel 2 wurde unter den Bedingungen von Beispiel 1 folgendes System der Polymerisation unter worfen :

400 ml Hexan, 0,5 g Copolymerisat aus Maleinsäureanhydrid und Vinylstearylether (wie in Beispiel 2), 22 g Vinylencarbonat, 22 g Vinylacetat, 6 g Butandiol-1,4-divinylether, 5 ml Diisopropylpercarbonat (40 % in Phthalat), 20 ml Polyethylenglykol (Molgewicht 400).

Die Polymerisationszeit betrug 3 Stunden bei 60° und eine Stunde bei 70°. Die Aufarbeitung erfolgte wie in Beispiel 5.

Ausbeute : 30 g (= 60 % der Theorie).

## Beispiel 7

Copolymerisat aus Vinylencarbonat, Vinylacetat und Butandiol-1,4-divinylether

In der Apparatur gemäß Beispiel 2 wurde unter den Bedingungen von Beispiel 1 folgendes System der Polymerisation unterworfen :

900 ml Paraffinöl, 0,1 g Copolymerisat aus Maleinsäureanhydrid und Vinylstearylether (wie in Beispiel 2), 43 g Vinylencarbonat, 43 g Vinylacetat, 20 g Butandiol-1,4-divinylether, 20 ml Dimethylsulfoxid, 4 ml Polyethylenglykol (Molgewicht 400), 2 g Azo-diisobuttersäurenitril.

Die Polymerisationszeit betrug eine Stunde bei 80° und 3 Stunden bei 90°.

Die Aufarbeitung erfolgte wie in Beispiel 1.

Ausbeute : 76 g (= 71 % der Theorie).

## Beispiel 8

Copolymerisat aus Vinylencarbonat, N-Vinylpyrrolidon und N,N-Methylenbisacrylamid

In der Apparatur gemäß Beispiel 2 wurde unter den Bedingungen von Beispiel 1 folgendes System der Polymerisation unterworfen :

900 ml Paraffinöl, 1 g Copolymerisat aus Maleinsäureanhydrid und Octadecen-(1) (wie in Beispiel 4), 36,4 g Vinylencarbonat, 46,9 g N-Vinylpyrrolidon, 16,6 g Methylenbisacrylamid, 100 ml Dimethylformamid, 2 g Azo-diisobuttersäurenitril.

Die Polymerisationszeit betrug eine Stunde bei 80° und 3 Stunden bei 90°.

Die Aufarbeitung erfolgte durch fünfstündiges Ausrühren mit Hexan zur Entfernung des Paraffinöles, fünfstündiges Ausrühren mit Methanol zur Entfernung der nicht umgesetzten Monomeren und des Dispergators und zweistündiges Ausrühren mit Aceton zur Entfernung der Restbestandteile. Anschließend wurde das Produkt 24 Stunden bei 50-60° im Vakuumtrockenschrank getrocknet.

Ausbeute : 65 g (= 65 % der Theorie).

Beispiel 9

Copolymerisat aus Vinylencarbonat, Methacrylsäuremethylester und Butandiol-1,4-divinylether

In der Apparatur gemäß Beispiel 2 wurde unter den Bedingungen von Beispiel 1 folgendes System der Polymerisation unterworfen :

900 ml Paraffinöl, 0,2 g Copolymerisat aus Maleinsäureanhydrid und Vinylstearylether (gemäß Beispiel 2), 43 g Vinylencarbonat, 50 g Methacrylsäuremethylester, 20 g Butandiol-1,4-divinylether, 4 ml Polyethylenglykol (Molgewicht 400), 2 g Azo-diisobuttersäurenitril.

Die Polymerisationszeit betrug eine Stunde bei 80° und 3 Stunden bei 90°.

Die Aufarbeitung erfolgte wie in Beispiel 8.

Ausbeute : 80 g (= 70 % der Theorie).

Beispiel 10

Copolymerisat aus Vinylencarbonat, N-Vinylpyrrolidon und Butandiol-1,4-divinylether

In der Apparatur von Beispiel 2 wurde unter den Bedingungen von Beispiel 1 folgendes System der Polymerisation unterworfen :

800 ml Paraffinöl, 1,5 g Copolymerisat aus Maleinsäureanhydrid und Octadecen-(1) (gemäß Beispiel 1), 43 g Vinylencarbonat, 55,5 g N-Vinylpyrrolidon, 52 g Butandiol-1,4-divinylether, 100 ml Ethylencarbonat, 1,5 g Azo-diisobuttersäurenitril.

Die Polymerisationszeit betrug 2 Stunden bei 80° und 2 Stunden bei 90°.

Die Aufarbeitung erfolgte wie in Beispiel 8.

Ausbeute : 92 g (= 61 % der Theorie).

Beispiel 11

Copolymerisat aus Vinylencarbonat, vinylpyrrolidon und Butandiol-1,4-divinylether

In der Apparatur von Beispiel 2 wurde unter den Bedingungen von Beispiel 1 folgendes System der Polymerisation unterworfen :

900 ml Paraffinöl, 0,2 g Copolymerisat aus Maleinsäureanhydrid und Vinylstearylether (gemäß Beispiel 2), 43 g Vinylencarbonat, 55,5 g N-Vinylpyrrolidon, 20 g Butandiol-1,4-divinylether, 4 ml Polyethylenglykol (Molgewicht 400), 3 g Azo-diisobuttersäurenitril (in 2 Portionen zugesetzt).

Die Polymerisationszeit betrug 30 Stunden bei 70°. Die erste Portion von 1,5 g Katalysator wurde zu Beginn, die zweite Portion von 1,5 g nach 15 Stunden Polymerisationszeit zugegeben.

Die Aufarbeitung erfolgte wie in Beispiel 8.

Ausbeute : 91 g (= 77 % der Theorie).

Beispiel 12

Copolymerisat aus Vinylencarbonat, N-Vinylpyrrolidon und N,N-Methylenbisacrylamid

In der Apparatur von Beispiel 2 wurde unter den Bedingungen von Beispiel 1 folgendes System der Polymerisation unterworfen :

900 ml Paraffinöl, 0,2 g Copolymerisat aus Maleinsäureanhydrid und Vinyl-stearylether (gemäß Beispiel 2), 37 g Vinylencarbonat, 48 g N-Vinylpyrrolidon, 15 g N,N-Methylenbisacrylamid, 106 ml Dimethylsulfoxid, 4 ml Polyethylenglykol (Molgewicht 400), 2 g Azo-diisobuttersäurenitril.

Die Polymerisationszeit betrug eine Stunde bei 80° und 4 Stunden bei 90°.

Die Aufarbeitung erfolgte wie in Beispiel 8.

Ausbeute : 79 g (79 % der Theorie).

Beispiel 13

Copolymerisat aus Vinylencarbonat, N-Vinylpyrrolidon und Trisacryloylperhydrotriazin

In der Apparatur von Beispiel 2 wurde unter den Bedingungen von Beispiel 1 folgendes System der Polymerisation unterworfen :

900 ml Paraffinöl, 0,4 g Copolymerisat aus Maleinsäureanhydrid und Vinylstearylether (gemäß Beispiel 2), 42 g Vinylencarbonat, 54,5 g N-Vinylpyrrolidon, 3,5 g Trisacryloylperhydrotriazin, 50 ml Diethylenglykoldimethylether, 2 g Azo-diisobuttersäurenitril.

Die Polymerisationszeit betrug 2 Stunden bei 80° und 2 Stunden bei 90°.

Die Aufarbeitung erfolgte wie in Beispiel 8.

Ausbeute : 69 g (= 69 % der Theorie).

Beispiel 14

Copolymerisat aus Vinylencarbonat, N-Vinylpyrrolidon und N,N'-Methylenbisacrylamid

In der Apparatur von Beispiel 2 wurde unter den Bedingungen von Beispiel 1 folgendes System der Polymerisation unterworfen :

900 ml Paraffinöl, 1 g Copolymerisat aus Maleinsäureanhydrid und Octadecen-(1) (gemäß Beispiel 4), 23 g Vinylencarbonat, 60 g N-Vinylpyrrolidon, 17 g N,N-Methylenbisacrylamid, 100 ml Dimethylformamid, 2 g Azo-diisobuttersäurenitril.

Die Polymerisationszeit betrug eine Stunde bei 80° und 3 Stunden bei 90°.

Die Aufarbeitung erfolgte wie in Beispiel 8.

Ausbeute : 55 g (= 55 % der Theorie).

Beispiel 15

Copolymerisat aus Vinylencarbonat, N-Vinylpyrrolidon und Divinylethylenharnstoff

In der Apparatur von Beispiel 2 wurde unter den Bedingungen von Beispiel 1 folgendes System der Polymerisation unterworfen :

900 ml Paraffinöl, 1 g Copolymerisat aus Maleinsäureanhydrid und Octadecen-(1) (gemäß Beispiel 1), 26 g Vinylencarbonat, 34 g N-Vinylpyrrolidon, 40 g Divinylethylenharnstoff, 100 ml Dimethylformamid, 100 ml Glycerin, 2 g Azo-diisobuttersäurenitril.

Die Polymerisationszeit betrug eine Stunde bei 80° und 3 Stunden bei 90°.

Die Aufarbeitung erfolgte wie in Beispiel 8 beschrieben.

Ausbeute : 92 g (= 92 % der Theorie).

Beispiel 16

Copolymerisat aus Vinylencarbonat, N-Vinylpyrrolidon und Divinylethylenharnstoff

In der Apparatur von Beispiel 2 wurde unter den Bedingungen von Beispiel 1 folgendes System der Polymerisation unterworfen :

900 ml Paraffinöl, 0,1 g Copolymerisat aus Maleinsäureanhydrid und Vinylstearylether (gemäß Beispiel 2), 26 g Vinylencarbonat, 34 g N-Vinylpyrrolidon, 40 g Divinylethylenharnstoff, 30 ml Dimethylformamid, 70 ml Glycerintriacetat, 2 g Azo-diisobuttersäurenitril.

Die Polymerisationszeit betrug drei Stunden bei 80° und eine Stunde bei 90°.

Die Aufarbeitung erfolgte durch achtstündiges Ausrühren mit Hexan und anschließendem einstündigen Ausrühren mit Aceton.

Ausbeute : 78 g (= 78 % der Theorie).

Schüttgewicht : 444 (g/l)

Schüttdichte : 2,2 (ml/g)

Quellung : 3,8 (ml/g) : (18 Stunden in Tetrahydrofuran bei 20 °C)

Porenvolumen : 0,52 ($cm^3$/g) (Quecksilberporosimetrie)

Mittlerer Porendurchmesser : 34 (nm)

Zur Bestimmung der Quellung wurde die Apparatur benutzt, die in der Dechema-Monographie Bd. 84 (1979) auf Seiten 73 und 74 beschrieben ist. Die Einwaage betrug 100 mg Träger bei 5 ml Tetrahydrofuran.

Beispiel 17

Copolymerisat aus Vinylencarbonat, N-Vinylpyrrolidon und Divinylethylenharnstoff

In der Apparatur von Beispiel 2 wurde unter den Bedingungen von Beispiel 1 folgendes System der Polymerisation unterworfen :

900 ml Paraffinöl, 0,1 g Copolymerisat aus Maleinsäureanhydrid und Vinylstearylether (gemäß Beispiel 2), 30,5 g Vinylencarbonat, 39,5 g N-Vinylpyrrolidon, 30 g Divinylethylenharnstoff, 20 ml Dimethylformamid, 80 ml Glycerintriacetat, 2 g Azo-diisobuttersäurenitril.

Die Polymerisationszeit betrug vier Stunden bei 80° und eine Stunde bei 90°.

Die Aufarbeitung erfolgte wie in Beispiel 16 beschrieben.

Ausbeute : 91 g (= 91 % der Theorie).

Schüttgewicht : 497 (g/l)

Schüttdichte : 2,0 (ml/g)

Quellung : 3,0 (ml/g) : (18 Stunden in Tetrahydrofuran bei 20 °C)

Porenvolumen : 0,47 ($cm^3$/g) (Quecksilberporosimetrie)

Mittlerer Porendurchmesser : 30 (nm)

Beispiel 18

Copolymerisat aus Vinylencarbonat, N-Vinylpyrrolidon und Divinylethylenharnstoff

In der in Beispiel 2 beschriebenen Apparatur wurde die folgende Copolymerisation durchgeführt: Zunächst wurden im Reaktionskolben 900 g Paraffinöl (dünnflüssig), 0,2 g Copolymerisat aus Maleinsäureanhydrid und Vinylstearylether gemäß Beispiel 2 vorgelegt. In einem Becherglas wurde dann ein Gemisch aus 30,6 g Vinylencarbonat, 39,4 g N-Vinylpyrrolidon, 30 g Divinylethylenharnstoff, 120 ml Dimethylformamid und 2 g Azo-diisobuttersäurenitril homogen verrührt und dann 60 ml entsalztes Wasser zugefügt. Diese Mischung wurde dem Reaktionskolben zugesetzt und unter Rühren der Ansatz auf 70° hochgeheizt, 1 Stunde bei dieser Temperatur gehalten und dann 4 Stunden bei 80° auspolymerisiert. Nach Abkühlen auf ca. 50° wurde das Copolymerisat abgesaugt, und dann in folgender Reihenfolge gewaschen: Heptan-Methanol-Aceton. Nach Trocknung unter Vakuum wurden 80 g (80 % der Theorie) Polymerisat erhalten.

Beispiel 19

Hydrolyse des Copolymerisates aus Vinylencarbonat, N-Vinylpyrrolidon und N,N'-Methylenbisacrylamid

In einem 3-Halskolben mit Rührer, Rückflußkühler und Thermometer wurden 30 g des obigen Copolymerisates gemäß Beispiel 14 in 150 ml Methanol und 50 ml 2 n-Natronlauge ca. 4 Stunden bei Rückflußtemperatur gerührt. Unter Abspaltung von Kohlendioxid wurde die Carbonatgruppe in eine Glykolgruppe überführt. Nach Neutralisation mit verdünnter Salzsäure wurde das Copolymerisat abgesaugt und mit Wasser/Methanol neutral gewaschen. Nach Trocknung im Vakuumschrank bei 60° wurden 22 g des hydrolysierten Copolymerisates erhalten.

Umsetzung mit Epichlorhydrin

20 g des oben genannten Produktes wurden in derselben Apparatur unter Rühren 5 Stunden bei 113-115° mit 150 ml Epichlorhydrin behandelt. Nach Absaugen des nicht umgesetzten Epichlorhydrins wurde mehrmals mit Aceton gewaschen und dann bei 50° im Vakuumschrank getrocknet. Es wurden 25 g eines oxiranhaltigen, schwach gelb gefärbten Copolymerisates erhalten. Die Analyse des Umsetzungsproduktes ergab 364 µMol/g an Epoxy-Einheiten.

II. Umsetzung der Perlpolymerisate mit biologisch aktiven Substanzen

Beispiel 20

Zu 3 Gramm eines nach Beispiel 1 hergestellten Trägers wurden 12 ml einer Trypsin-Lösung (6,25 mg/ml, 345 U/ml), die $1{,}6 \times 10^{-2}$ molar an Benzamidin und 1 molar an Kaliumphosphat (Puffer) war und einen pH-Wert von 7,8 aufwies, gegeben. Nach der Fixierung über Nacht (16 h) wurden die Perlen mit 1 molarer Kochsalzlösung und mit Puffer-Lösung gründlich gewaschen. Die Ausbeute an nutschenfeuchtem Material betrug 8,2 Gramm mit 270 Units/g, gemessen am Autotitrator bei 37 °C und einem pH-Wert von 8,1 mit N'-Benzoyl-L-argininäthylesterhydrochlorid (BAEE) als Substrat. Bezogen auf Trockengewicht waren das 745 Units/g. Nach Bilanzierung von Ausgangs- und Waschwasseraktivität blieb eine Fixierungsausbeute (= Aktivität auf Träger: angebotene Aktivität) von 54 %. Der $\eta$-Wert betrug 0,56 ($\eta$ = gefundene Aktivität/(angebotene Aktivität minus Waschwasser-Aktivität)).

Beispiel 21

Zu 2 Gramm eines nach Beispiel 1 hergestellten Trägers wurden 8 ml einer Cephalosporinase-Lösung gegeben, die 1 500 Units/ml enthielt, wobei 1 Unit die Bildung von 1 µMol geöffnetem Laktamring pro Minute bei einem pH-Wert von 7,8 und 37 °C entsprach. Die Reaktion wurde am Autotitrator über die Natronlaugezugabe verfolgt.

Nach einem Tag Fixierung wurde das Trägermaterial wie im vorhergehenden Beispiel gewaschen; man erhielt ca. 7,5 Gramm nutschenfeuchtes hochaktives Material mit mind. 1 300 Units/g bzw. 5 000 Units/g bezogen auf Trockenmasse. Die Fixierungsausbeute betrug nach Bilanzierung 81 %. ($\eta$ = 1).

Beispiel 22

Zu 0,1 Gramm Träger nach Beispiel 15 wurden 0,4 ml D-Aminosäureoxidase-Lösung mit 7 Units/ml bzw. 0,6 Units/mg Protein gegeben, gemessen mit Cephalosporin C als Substrat über die Freisetzung von $H_2O_2$ nach DE-OS 22 19 454 bei einem pH-Wert von 8,1 und einer Temperatur von 37 °C in 0,1 molarem Pyrophosphatpuffer.

Nach 16 Stunden Bindungszeit wurde das trägerfixierte Enzym gewaschen und die Aktivität

bestimmt. Es wurden 1,25 Units/g nutschenfeuchtes Material bzw. 5,4 Units/g trockenes Material bestimmt. Die Fixierungsausbeute betrug 19 % ($\eta$ = 0,24).

Beispiel 23

Zu 1 g eines nach Beispiel 8 hergestellten Trägers wurden 22 mg α-Amylase-Inhibitor (Tendamistat), das sind 374 000 int. Inhibitoreinheiten = 100 %, gelöst in 5 ml 1 molarer Natriumbikarbonat-Lösung gegeben. Nach 16 Stunden Bindungszeit wurde das Trägermaterial mit dem fixierten Enzym gewaschen. Das Waschwasser enthielt noch 60 % der eingesetzten Aktivität.

Der trägergebundene Inhibitor war in der Lage, α-Amylase zu binden.

Beispiel 24

Zu 0,2 g des oxiranhaltigen Copolymerisates gemäß Beispiel 19 wurden 1 200 µl einer Penicillin-Acylase-Lösung (40 mg/ml ; 287 U/ml) in 1 molarem Kaliumphosphatpuffer mit einem pH-Wert von 8,0 zugesetzt. Nach einer Fixierungszeit von 72 Stunden wurde der Träger mit 1-molarer Kochsalzlösung und vorstehender Pufferlösung gut ausgewaschen. Die Ausbeute an nutschenfeuchtem Material betrug 840 mg mit 315 U/g, gemessen mit dem Autotitrator bei 37° und einem pH-Wert von 7,8 mit penicillinsaurem Kalium als Substrat. Umgerechnet auf Trockengewicht erhielt man 1 365 U/g. Nach Bilanzierung von Ausgangs- und Waschwasseraktivität ergab sich eine Fixierungsausbeute von 79 % (Aktivität auf Trägerangebotene Aktivität). Der $\eta$-Wert betrug 0,81.

**Patentansprüche** (für die Vertragsstaaten : BE, CH, DE, FR, GB, IT, LI, LU, NL, SE)

1. Polymerisat, bestehend aus Einheiten, die sich von Vinylencarbonat oder dessen durch einen einwertigen Kohlenwasserstoffrest mit bis zu 8 Kohlenstoffatomen substituiertes Derivat und mindestens einem weiteren damit copolymerisierbaren Monomeren ableiten, wobei die mittlere Teilchengröße der Polymerteilchen im Bereich von 20 bis 800 µm liegt, dadurch gekennzeichnet, daß die Polymerteilchen im wesentlichen perlförmige Gestalt und einen mittleren Porendurchmesser von 5 bis 1 000 nm aufweisen.

2. Polymerisat nach Anspruch 1, dadurch gekennzeichnet, daß der mittlere Porendurchmesser 10 bis 500 nm beträgt.

3. Polymerisat nach Anspruch 1 oder 2, dadurch gekennzeichnet, daß die Menge an Vinylencarbonat-Einheiten 5 bis 90 Mol.-%, bezogen auf das Gesamtpolymere, beträgt.

4. Polymerisat nach einem oder mehreren der Ansprüche 1 bis 3, dadurch gekennzeichnet, daß es neben den Vinylencarbonat-Einheiten noch solche Einheiten enthält, die sich von Monomeren mit hydrophilen Gruppen ableiten.

5. Polymerisat nach Anspruch 4, dadurch gekennzeichnet, daß es infolge des Einbaues von vernetzenden Monomer-Einheiten vernetzt ist.

6. Polymerisat nach Anspruch 5, dadurch gekennzeichnet, daß die Menge an vernetzender Monomer-Einheit so groß ist, daß das Polymerisat in Tetrahydrofuran bis auf das 14-fache seines ursprünglichen Schüttvolumens aufquillt.

7. Polymerisat nach einem oder mehreren der Ansprüche 1 bis 6, dadurch gekennzeichnet, daß es einen Spacer kovalent gebunden enthält.

8. Verfahren zur Herstellung der Polymerisate nach einem oder mehreren der Ansprüche 1 bis 7 durch Polymerisieren von Vinylencarbonat oder dessen durch einen einwertigen Kohlenwasserstoffrest mit bis zu 8 Kohlenstoffatomen substituiertes Derivat mit mindestens einem mit Vinylencarbonat copolymerisierbaren Monomeren in einem flüssigen Dispergiermedium, das unter den Polymerisationsbedingungen die Monomeren und das Polymerisat nicht löst, in Gegenwart eines radikalisch wirksamen Initiators und eines Dispersionsstabilisators, dadurch gekennzeichnet, daß als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen α-Olefin mit 8 bis 30 C-Atomen verwendet wird.

9. Verfahren nach Anspruch 8, dadurch gekennzeichnet, daß der Dispersionsstabilisator in Mengen von 0,005 bis 10 Gew.-%, bezogen auf das Monomerengemisch, verwendet wird.

10. Verfahren nach Anspruch 8 und/oder 9, dadurch gekennzeichnet, daß der Dispersionsstabilisator ein alternierendes Copolymerisat ist.

11. Verfahren nach einem oder mehreren der Ansprüche 8 bis 10, dadurch gekennzeichnet, daß der RSV-Wert des als Dispersionsstabilisator eingesetzten Copolymerisates zwischen 0,01 und 1,0 [dl/g] (gemessen in 0,6 %iger Lösung in Toluol bei 25 °C) liegt.

12. Verfahren nach einem oder mehreren der Ansprüche 8 bis 11, dadurch gekennzeichnet, daß der Vinylalkyläther Vinylstearyläther und das längerkettige α-Olefin Octadecen (1) ist.

13. Verfahren nach einem oder mehreren der Ansprüche 8 bis 12, dadurch gekennzeichnet, daß als flüssige Dispergiermittel Kohlenwasserstoffe mit 6 bis 20 C-Atomen oder Paraffinöl dünnflüssig eingesetzt werden.

14. Verfahren nach einem oder mehreren der Ansprüche 8 bis 13, dadurch gekennzeichnet, daß das Polymerisationssystem zur Erhöhung der Porosität des Perlpolymerisates Stoffe enthält, die sich in den Monomeren gut lösen oder mit ihnen mischbar sind und im Dispergiermedium praktisch unlöslich sind.

15. Verwendung der Polymerisate nach einem oder mehreren der Ansprüche 1 bis 7, gegebenenfalls nach Umsatz mit Spacern, zur Herstellung trägergebundener, biologisch aktiver Substanzen.

16. Verwendung nach Anspruch 15, dadurch gekennzeichnet, daß die biologisch aktiven Substanzen Enzyme sind.

17. Verwendung nach Anspruch 15 oder 16, dadurch gekennzeichnet, daß vor der Umsetzung mit dem Spacer eine Verseifung der Carbonatgruppen erfolgt.

18. Verwendung nach Anspruch 17, dadurch gekennzeichnet, daß der Spacer Epichlorhydrin darstellt.

**Patentansprüche** (für den Vertragsstaat AT)

1. Verfahren zur Herstellung von Polymerisaten, bestehend aus Einheiten, die sich vom Vinylencarbonat oder dessen durch einen einwertigen Kohlenwasserstoffrest mit bis zu 8 Kohlenstoffatomen substituiertes Derivat und mindestens einem weiteren damit copolymerisierbaren Monomeren ableiten, durch Polymerisieren von Vinylencarbonat oder dessen durch einen einwertigen Kohlenwasserstoffrest mit bis zu 8 Kohlenstoffatomen substituiertes Derivat mit mindestens einem Vinylencarbonat copolymerisierbaren Monomeren in einem flüssigen Dispergiermedium, das unter den Polymerisationsbedingungen die Monomeren und das Polymerisat nicht löst, in Gegenwart eines radikalisch wirksamen Initiators und eines Dispersionsstabilisators, dadurch gekennzeichnet, daß als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen α-Olefin mit 8 bis 30 C-Atomen verwendet wird.

2. Verfahren nach Anspruch 1, dadurch gekennzeichnet, daß der Dispersionsstabilisator in Mengen von 0,005 bis 10 Gew.-%, bezogen auf das Monomerengemisch, verwendet wird.

3. Verfahren nach Anspruch 1 und 2, dadurch gekennzeichnet, daß der Dispersionsstabilisator ein alternierendes Copolymerisat ist.

4. Verfahren nach Anspruch 1 bis 3, dadurch gekennzeichnet, daß der RSV-Wert des als Dispersionsstabilisator eingesetzten Copolymerisates zwischen 0,01 und 1,0 [dl/g] (gemessen in 0,6 %iger Lösung in Toluol bei 25 °C) liegt.

5. Verfahren nach Anspruch 1 bis 4, dadurch gekennzeichnet, daß der Vinylalkyläther Vinylstearyläther und das längerkettige α-Olefin Octadecen (1) ist.

6. Verfahren nach Anspruch 1 bis 5, dadurch gekennzeichnet, daß als flüssige Dispergiermittel Kohlenwasserstoffe mit 6 bis 20 C-Atomen oder Paraffinöl dünnflüssig eingesetzt werden.

7. Verfahren nach Anspruch 1 bis 6, dadurch gekennzeichnet, daß das Polymerisationssystem zur Erhöhung der Porosität des Perlpolymerisates Stoffe enthält, die sich in den Monomeren gut lösen oder mit ihnen mischbar sind und im Dispergiermittel praktisch unlöslich sind.

8. Verfahren nach Anspruch 1 bis 7, dadurch gekennzeichnet, daß mit dem Vinylcarbonat mindestens zwei Monomere copolymerisiert werden, die Monomere mit hydrophilen Gruppen und/oder Monomere mit vernetzenden Gruppen darstellen.

9. Verwendung der nach Anspruch 1 bis 8 erhaltenen Polymerisate, gegebenenfalls nach Umsatz mit Spacern, zur Herstellung trägergebundener, biologisch aktiver Substanzen.

10. Verwendung nach Anspruch 9, dadurch gekennzeichnet, daß die biologisch aktiven Substanzen Enzyme sind.

11. Verwendung nach Anspruch 9 und 10, dadurch gekennzeichnet, daß vor der Umsetzung mit dem Spacer eine Verseifung der Carbonatgruppen erfolgt und der Spacer Epichlorhydrin darstellt.

**Claims** (for the Contracting states : BE, CH, DE, FR, GB, IT, LI, LU, NL, SE)

1. A polymer composed of units which are derived from vinylene carbonate or a derivative thereof which is substituted by a monovalent hydroxarbon radical having up to 8 carbon atoms, and of at least one other monomer which can be copolymerized with it, the mean particle size of the polymer particles being in the range from 20 to 800 μm, wherein the polymer particles have an essentially spherical shape and a mean pore diameter of 5 to 1 000 nm.

2. The polymer as claimed in claim 1, wherein the mean pore diameter is 10 to 500 nm.

3. The polymer as claimed in either of claims 1 or 2, wherein the amount of vinylene carbonate units is 5 to 90 mole-% relative to the total polymer.

4. The polymer as claimed in any of claims 1 to 3, which, in addition to the vinylene carbonate units, contains units which are derived from monomers having hydrophilic groups.

5. The polymer as claimed in claim 4, which, as a consequence of incorporation of crosslinking monomer units, is crosslinked.

6. The polymer of claim 5, wherein the amount of crosslinking monomer units is selected such that the bead polymer swells in tetrahydrofurane by up to 14 times its original bulk volume.

7. The polymer as claimed in any of claims 1 to 6 which contains a spacer covalently bonded.

8. A process for the preparation of the polymer as claimed in any of claims 1 to 7, by polymerization of vinylene carbonate or a derivative thereof which is substituted by a monovalent hydrocarbon radical having up to 8 carbon atoms with at least one monomer which can be copolymerized with vinylene carbonate, in a liquid dispersing medium, which does not dissolve the monomers and the polymer under the polymerization conditions, in the presence of a radical initiator and a dispersion stabilizer, which comprises using, as the dispersion stabilizer, a copolymer of maleic anhydride and a vinyl alkyl ether having 6 to 30 carbon atoms in the carboxylic acid group, or a relatively long-chain α-olefin having 8 to 30 carbon atoms.

9. The process as claimed in claim 8, wherein in the dispersion stabilizer is used in amounts of 0.005 to 10 % by weight relative to the mixture of monomers.

10. The process as claimed in either of claims 8 or 9, wherein the dispersion stabilizer in an alternating copolymer.

11. The process as claimed in any of claims 8 to 10, wherein the RSV value of the copolymer employed as a dispersion stabilizer is between 0.01 and 1.0 [dl/g] (measured in 0.6 % strength solution in toluene at 25 °C).

12. The process as claimed in any of claims 8 to 11, wherein the vinyl alkyl ether is vinyl stearyl ether and the relatively long-chain α-olefin is 1-ocetadecene.

13. The process as claimed in any of claims 8 to 12, wherein hydrocarbons having 6 to 20 carbons atoms, or low viscosity liquid paraffin is employed as the liquid dispersing agent.

14. The process as claimed in any of claims 8 to 13, wherein, in order to increse the porosity of the bead polymer, the polymerization system contains materials which are readily soluble in or miscible with the monomers and are virtually insoluble in the dispersing medium.

15. The use of the polymer as claimed in any of claims 1 to 7, where appropriate after reaction with spacers, for the preparation of carrier-bound biologically active substances.

16. The use as claimed in claim 15, wherein the biologically active substances are enzymes.

17. The use as claimed in either of claims 15 or 16, wherein, before reaction with the spacer, the carbonate groups are hydrolyzed.

18. The use as claimed in claim 17, wherein the spacer is epichlorohydrin.

**Claims** (for the Contracting State AT)

1. A process for the preparation of a polymer composed of units which are derived from vinylene carbonate or a derivative thereof which is substituted by a monovalent hydrocarbon radical having up to 8 carbon atoms, and of at least one other monomer which can be copolymerized with it, by polymerization of vinylene carbonate or a derivative thereof which is substituted by a monovalent hydrocarbon radical having up to 8 carbon atoms with at least one monomer which can be copolymerized with vinylene carbonate, in a liquid dispersing medium, which does not dissolve the monomers and the polymer under the polymerization conditions, in the presence of a radical initiator and a dispersion stabilizer, which comprises using, as the dispersion stabilizer, a copolymer of maleic anhydride and a vinyl alkyl ether having 6 to 30 carbon atoms in the alkyl group, or a vinyl ester having 6 to 30 carbon atoms in the carboxylic acid group, or a relatively long-chain α-olefin having 8 to 30 carbon atoms.

2. The process as claimed in claim 1, wherein the dispersion stabilizer is used in amounts of 0.005 to 10 % by weight relative to the mixture of monomers.

3. The process as claimed in either of claims 1 or 2, wherein the dispersion stabilizer is an alternating copolymer.

4. The process as claimed in any of claims 1 to 3, wherein the RSV value of the copolymer employed as a dispersion stabilizer is between 0.01 and 1.0 [dl/g] (measured in 0.6 % strength solution in toluene at 25 °C).

5. The process as claimed in any of claims 1 to 4, wherein the vinyl alkyl ether is vinyl stearyl ether and the relatively long-chain α-olefin is 1-octadecene.

6. The process as claimed in any of claims 1 to 5, wherein hydrocarbons having 6 to 20 carbon atoms, or low viscosity liquid paraffin is employed as the liquid dispersing agent.

7. The process as claimed in any of claims 1 to 6, wherein, in order to increase the porosity of the bead polymer, the polymerization system contains materials which are readily soluble in or miscible with the monomers and are virtually insoluble in the dispersing medium.

8. The process as claimed in any of claims 1 to 7, wherein the vinyl carbonate is copolymerized with at least two other monomers, which monomers contain hydrophilic groups and/or crosslinking groups.

9. The use of the polymer as claimed in any of claims 1 to 8, where appropriate after reaction with spacers, for the preparation of carrier-bound biologically active substances.

10. The use as claimed in claim 9, wherein the biologically active substances are enzymes.

11. The use as claimed in either of claims 9 or 10, wherein, before reaction with the spacer, the carbonate groups are hydrolyzed, and wherein the spacer is epichlorohydrin.

**Revendications** (pour les Etats contractants : BE, CH, DE, FR, GB, IT, LI, LU, NL, SE)

1. Polymère, consistant en des unités qui dérivent du carbonate de vinylène ou de son dérivé de substitution par un reste hydrocarboné monovalent comportant jusqu'à 8 atomes de carbone et d'au moins un autre monomère copolymérisable avec lui, la grosseur moyenne des particules du polymère se situant entre 20 et 800 µm, polymère caractérisé en ce que les particules de ce polymère ont essentiellement la forme de perles et présentent un diamètre moyen des pores de 5 à 1 000 nm.

2. Polymère selon la revendication 1, caractérisé en ce que le diamètre moyen des pores est de 10 à 500 nm.

3. Polymère selon la revendication 1 ou 2, caractérisé en ce que la proportion des motifs carbonates de vinylène est de 5 à 90 moles%, par rapport au polymère global.

4. Polymère selon une ou plusieurs des revendications 1 à 3, caractérisé en ce que, en plus des motifs carbonates de vinylène, il contient encore des motifs qui dérivent de monomères comportant des groupes hydrophiles.

5. Polymère selon la revendication 4, caractérisé en ce qu'il est réticulé par suite de l'incorporation de motifs monomères à rôle de réticulation.

6. Polymère selon la revendication 5, caractérisé en ce que la proportion de motifs de monomères à rôle de réticulation est telle que le polymère subit dans le tétrahydrofuranne un gonflement pouvant aller jusqu'à 14 fois son volume massique d'origine.

7. Polymère selon une ou plusieurs des revendications 1 à 6, caractérisé en ce qu'il contient un « Spacer » (terme de pontage intercalé) lié par covalence.

8. Procédé pour préparer les polymères selon une ou plusieurs des revendications 1 à 7 par polymérisation du carbonate de vinylène ou de son dérivé de substitution par un reste hydrocarbure monovalent ayant jusqu'à 8 atomes de carbone avec au moins un monomère copolymérisable avec le carbonate de vinylène dans un milieu liquide de dispersion, qui, dans les conditions de polymérisation, ne dissout pas les monomères et le polymère, en présence d'un amorceur à rôle radicalaire, et d'un stabilisant de la dispersion, procédé caractérisé en ce qu'on utilise comme stabilisant de la dispersion un copolymère de l'anhydride de l'acide maléique et d'un éther de vinyle et d'alkyle comportant 6 à 30 atomes de carbone dans le groupe alkyle ou d'un ester vinylique comportant 6 à 30 atomes de carbone dans le groupe acide carboxylique, ou d'une alpha-oléfine à longue chaîne comportant 8 à 30 atomes de carbone.

9. Procédé selon la revendication 8, caractérisé en ce qu'on utilise le stabilisant de la dispersion en des proportions de 0,005 à 10 % en poids, par rapport au mélange des monomères.

10. Procédé selon la revendication 8 et/ou 9, caractérisé en ce que le stabilisant de la dispersion est un copolymère alterné.

11. Procédé selon une ou plusieurs des revendications 8 à 10, caractérisé en ce que la valeur de la viscosité spécifique réduite du copolymère utilisé comme stabilisant de la dispersion se situe entre 0,01 et 1,0 [dl/g] (la mesure étant effectuée dans le cas d'une solution à 0,6 % dans du toluène à 25 °C).

12. Procédé selon une ou plusieurs des revendications 8 à 11, caractérisé en ce que l'éther d'alkyle et de vinyle est l'éther de stéaryle et de vinyle, et l'alpha-oléfine à longue chaîne est l'octadécène-(1).

13. Procédé selon une ou plusieurs des revendications 8 à 12, caractérisé en ce qu'on utilise comme agent liquide de dispersion des hydrocarbures comportant 6 à 20 atomes de carbone ou de l'huile de paraffine fluide.

14. Procédé selon une ou plusieurs des revendications 8 à 13, caractérisé en ce que, pour augmenter la porosité de la substance polymérisée en perles, le système de polymérisation contient des substances qui se dissolvent bien dans les monomères ou sont miscibles à eux et sont pratiquement insolubles dans le milieu de dispersion.

15. Utilisation du polymère selon une ou plusieurs des revendications 1 à 7, éventuellement après réaction avec des « Spacers » (termes d'espacement et de pontage) pour préparer des substances biologiquement actives fixées sur un support.

16. Utilisation selon la revendication 15, caractérisée en ce que les substances biologiquement actives sont des enzymes.

17. Utilisation selon la revendication 15 ou 16, caractérisée en ce que, avant la réaction avec le « Spacer », on effectue une saponification des groupes carbonates.

18. Utilisation selon la revendication 17, caractérisée en ce que le « Spacer » est l'épichlorhydrine.

**Revendications** (pour l'Etat contractant AT)

1. Procédé pour préparer des polymères consistant en des motifs qui dérivent du carbonate de vinylène ou d'un dérivé de ce composé substitué par un reste hydrocarboné monovalent comportant jusqu'à 8 atomes de carbone et d'au moins un autre monomère copolymérisable avec lui, par polymérisation du carbonate de vinylène ou de son dérivé de substitution par un reste hydrocarboné monovalent comportant jusqu'à 8 atomes de carbone avec au moins un monomère copolymérisable avec le carbonate de vinylène dans un milieu liquide de dispersion qui, dans les conditions de polymérisation,

ne dissout pas les monomères et le polymère, en présence d'un amorceur à rôle radicalaire et d'un stabilisant de dispersion, procédé caractérisé en ce qu'on utilise comme stabilisant de la dispersion un copolymère de l'anhydride d'acide maléique et d'un éther de vinyle et d'alkyle comportant 6 à 30 atomes de carbone dans le groupe alkyle ou d'un ester vinylique comportant 6 à 30 atomes de carbone dans le groupe acide carboxylique ou d'une alpha-oléfine à longue chaîne comportant 8 à 30 atomes de carbone.

2. Procédé selon la revendication 1, caractérisé en ce qu'on utilise le stabilisant de la dispersion en des proportions de 0,005 à 10 % en poids, par rapport au mélange des monomères.

3. Procédé selon les revendications 1 et 2, caractérisé en ce que le stabilisant de la dispersion est un copolymère alterné.

4. Procédé selon l'une des revendications 1 à 3, caractérisé en ce que la valeur de la viscosité spécifique réduite du copolymère utilisé comme stabilisant de la dispersion se situe entre 0,01 et 1,0 [dl/g] (mesure effectuée dans le cas d'une solution à 0,6 % dans du toluène à 25 °C).

5. Procédé selon l'une des revendications 1 à 4, caractérisé en ce que l'éther de vinyle et d'alkyle est l'éther de vinyle et de stéaryle et l'alpha-oléfine à longue chaîne est l'octadécène-(1).

6. Procédé selon l'une des revendications 1 à 5, caractérisé en ce qu'on utilise comme agents liquides de dispersion les hydrocarbures ayant 6 à 20 atomes de carbone ou de l'huile de paraffine fluide.

7. Procédé selon l'une des revendications 1 à 6, caractérisé en ce que, pour augmenter la porosité du polymère en perles, le système de polymérisation contient des substances qui se dissolvent bien dans les monomères ou sont miscibles à eux et qui sont pratiquement insolubles dans le milieu de dispersion.

8. Procédé selon l'une des revendications 1 à 7, caractérisé en ce qu'on copolymérise, avec le carbonate de vinylène, au moins deux monomères qui sont des monomères comportant des groupes hydrophiles et/ou des monomères comportant des groupes à rôle de réticulation.

9. Utilisation des polymères, obtenus selon l'une des revendications 1 à 8, éventuellement après réaction avec des « Spacers » (termes d'espacement et de pontage), pour préparer des substances biologiquement actives fixées sur un support.

10. Utilisation selon la revendication 9, caractérisée en ce que les substances biologiquement actives sont des enzymes.

11. Utilisation selon les revendications 9 et 10, caractérisée en ce que, avant la réaction avec le « Spacer », on effectue une saponification des groupes carbonates et en ce que l'épichlorhydrine constitue le « Spacer ».